# EUROPÄISCHE PATENTSCHRIFT

(11) **EP 3 310 896 B1**
(45) Veröffentlichungstag und Bekanntmachung des Hinweises auf die Patenterteilung: **06.01.2021**
(21) Anmeldenummer: 16750605.4
(22) Anmeldetag: 15.06.2016
(51) Int. Cl.: C12M 1/00, C12N 5/00, C12N 11/06, C12N 11/08

(54) **BIOAKTIVES BESCHICHTUNGSMATERIAL**
BIOACTIVE COATING MATERIAL
MATÉRIAU DE REVÊTEMENT BIOACTIF

(30) Priorität: 16.06.2015 DE 102015109599
(43) Veröffentlichungstag der Anmeldung: 25.04.2018
(73) Patentinhaber: Zetascience GmbH, 01307 Dresden (DE)
(72) Erfinder: FREUDENBERG, Uwe, 01328 Dresden (DE)
(74) Vertreter: Sperling, Thomas
(86) Internationale Anmeldenummer: PCT/DE2016/100273
(87) Internationale Veröffentlichungsnummer: WO 2016/202329

(56) Entgegenhaltungen:
- EP-A1- 1 616 939
- US-A1- 2010 304 427
- US-A1- 2012 052 580

## Beschreibung

Die Erfindung betrifft ein Verfahren zur Beschichtung von Zellkulturträgern aus Kunststoff unter Verwendung eines bioaktiven Beschichtungsmaterials zur Beschichtung von Kunststoffmaterialien für Zellkulturen, vorzugsweise Kunststoffeinwegmaterialien, zum Beispiel aus Polystyren (TCP) als Kunststoff. Insbesondere betrifft die Erfindung dabei ein Verfahren unter Verwendung eines zellinstruktiven Beschichtungsmaterials für die Beschichtung von Zellkulturmaterialien aus Kunststoff, zum Beispiel Polystyren. Die Erfindung eröffnet neue Möglichkeiten für *in situ* Beschichtungen in Forschung und Biotechnologie.

Für Zellkulturanwendungen im Bereich der Forschung, der Biotechnologie und der Medizin werden derzeitig massenhaft Kunststoffeinwegartikel genutzt. Dabei besteht bei der Mehrzahl der Anwendungen ein Zellkontakt mit der Materialoberfläche und somit eine sogenannte adhärente Zellkultur. Zellinstruktiv bedeutet in diesem Zusammenhang ein Signal, welches die Zelladhäsion und Zellschicksalsentscheidungen wie Migration, Proliferation und Differenzierung der Zellen beeinflusst.

Zellkultureinwegprodukte werden in großer, stetig wachsender Zahl in bedeutsamen Technologiefeldern eingesetzt. Im Jahr 2009 bestand ein globales Marktvolumen für Zellkulturplatten von etwa 500 Millionen US-Dollar mit dem wirtschaftlichen Schwerpunkt in USA, Europa und Japan, wobei aufgrund des dynamischen Wachstums im Biotechnologie- und Life Science Bereich jährliche Zuwachsraten von 100 % bis zum Jahr 2015 diskutiert werden. Zellkulturplatten basieren derzeitig in der Mehrzahl auf sogenannten Tissue culture plastic (TCP), das heißt Polystyren-(PS)-Materialien, die zur Verbesserung der Oberflächeneigenschaften, speziell zur Stimulation der Zelladhäsion, funktionalisiert werden. Zuletzt wurden dafür hauptsächlich zwei verschiedene Konzepte angewandt: (I) chemische Oberflächenmodifizierungen und (II) Beschichtungen mit aus natürlichen Quellen isolierten Proteinen der extrazellulären Matrix (ECM).

Zur ersten Gruppe (I) sind die am Markt EU- beziehungsweise weltweit etablierten plasmafunktionalisierten TCP-Produkte zur Verbesserung der Benetzbarkeit und auch kovalent oder physisorptiv gekoppelte, adhäsive Beschichtungen zu zählen. Es werden dabei einerseits gezielt funktionelle Gruppen, zum Beispiel dauerhaft kationisch oder anionisch geladene Oberflächen, zum Beispiel BD PureCoat Amine/Carboxyl; BD Bioscience, und andere polare Gruppen, zum Beispiel Greiner CELLSTAR®, Corning® CellBIND®, in die Materialoberfläche eingeführt oder polykationische Beschichtungen, zum Beispiel Poly-D oder Poly-L-Lysin, zum Beispiel Nunclon®, BD BioCoat™ und Corning® Poly(D-Lysin)-coatings, aufgebracht, die unter Anwendungsbedingungen die Anbindung von ECM-Proteinen und somit indirekt die Zelladhäsion verbessern und weitere Zellschicksalsentscheidungen beeinflussen.

Die zweite Gruppe (II) kommerziell verfügbarer Beschichtungen besteht aus von biologischen Quellen isolierten ECM-Proteinen, zum Beispiel Kollagen, Fibronektin oder Laminin. Beispiele für solche Produkte sind Greiner CELLCOAT®, Millipore Millicoat™ oder BD BioCoat™. Darüber hinaus ist eine erste Anwendung dezellularisierter extrazellulärer Matrices von Endothelzellen, zum Beispiel von der Firma Novamed Ltd.-Coatings, bekannt.

Die erste Gruppe (I) von Funktionalisierungskonzepten dient der Verbesserung der Anhaftung zellsezernierter Proteine und anderer Matrixbestandteile an die Kunststoffoberflächen und kann somit indirekt zur Förderung der Zelladhäsion und zur Kontrolle von Zellschicksalsentscheidungen beitragen. Ein Vorteil dieser Beschichtungen liegt darin, dass das Produkt keine biologischen Komponenten enthält, so dass die Eigenschaften gut kontrollierbar und die Produkte gut lager- und sterilisierbar sind.

Die zweite Gruppe (II) verwendet aus biologischen Quellen isolierte Proteine, die spezifische Peptidsequenzen enthalten, die durch zelluläre Rezeptoren gebunden werden und so die Zelladhäsion oder weitere Zellschicksalsentscheidungen kontrollieren. Der Vorteil dieser Präparationen besteht darin, dass die Zellen direkt mit den Proteinbeschichtungen in Wechselwirkung treten.

Kommerziell etablierte bioaktive Beschichtungen beruhen jedoch zunehmend auf dem Einsatz von an Oberflächen gebundenen Peptidsequenzen. Diese werden zumeist kovalent auf TCP verankert, zum Beispiel bei CORNING® Synthemax™ und Biosciences® BioPure™, wobei inzwischen zumindest ein Beispiel existiert, wo Adhäsionspeptide direkt beim Endanwender adsorptiv über einen Anker aus hydrophoben Aminosäuren angekoppelt werden können, so bei Advanced Biomatrix PEPTITE 2000®. Der Vorteil dieser Beschichtungen liegt in der Nutzung von vollsynthetischen Adhäsionspeptiden zur Vermittlung der Zelladhäsion, wobei die kommerzielle Einführung bei verschiedenen weltweiten Marktführern für Zellkulturartikel das Potenzial derartiger bioadhäsiver Beschichtungen zeigt.

Bei chemischen Oberflächenmodifizierungen zur vermehrten Anbindung zellsekretierter Proteine erlaubt die lediglich indirekte Förderung der Zellanhaftung kaum eine Kontrolle über die finale Stärke der Zelladhäsivität oder die Art der Modulation von weiteren Zellschicksalsentscheidungen. Zudem können derartige Funktionalisierungen nicht selbst vom Endanwender durchgeführt werden, so dass auf eine bezüglich der Geometrie beschränkte Auswahl vom Hersteller angebotener Zellkulturartikel zurückgegriffen werden muss.

In der Druckschrift Yoo, H.Y et al., "Recombinant Mussel Coating Protein Fused with Cell Adhesion Recognition Motif Enhanced Cell Proliferation", Biotechnology and Bioprocess Engineering 20, S. 211 bis 217, 2015 ist eine haltbare bioaktive Beschichtung einer biomedizinischen Substratoberfläche aus Polystyren (TCP) beschrieben. Um eine solche Beschichtung zu erreichen, wird das Fusionsprotein Fp-1-RGD genetisch konstruiert und in *Escherichia coli* hergestellt. Nach Beschichtung eines TCP-Zellkulturträgers (TCP) mit Fp-1-RGD zeigte dieser eine bessere Zellproliferation von Präosteoblasten im Vergleich zu unbeschichteten TCP-Zellkulturträgern.

Die Nachteile von Beschichtungen mit aus natürlichen Quellen isolierten ECM-Proteinen, also Proteinen der extrazellulären Matrix, liegen in den aufgrund der biologischen Diversität nur begrenzt kontrollierbaren Beschichtungseigenschaften und der Möglichkeit, dass biologisch aktive Signalmoleküle, wie zum Beispiel Hormone, Neurotransmitter, Zytokine, Wachstumsfaktoren und Chemokine, über die Präparation in die Zellkultur verschleppt werden und somit die eigentlich zu untersuchenden Parameter beeinflusst werden können. Außerdem sind derartige Produkte hinsichtlich Lagerung und Sterilisierbarkeit problematisch. Bei der letzten Gruppe von bioaktiven Beschichtungen mit synthetischen Adhäsionspeptiden besteht die technologisch bedingte Einschränkung der Variabilität der Funktionalisierung auf bestimmte Peptidsequenzen und physikochemische Oberflächeneigenschaften, welche eine gezielte Abstufung der Zelladhäsion und anderer Zellschicksalsentscheidungen nicht möglich machen. Es ist davon auszugehen, dass insbesondere in komplexen Zellkulturmedien eine ausreichende Verankerung der Peptide nicht erreicht werden kann, das heißt, dass die Peptide mitsamt ihrer hydrophoben Ankermoleküle relativ leicht von der TCP-Oberfläche verdrängt werden und somit keine ausreichende Stabilität für die Zelladhäsion vermitteln. Neben diesen Nachteilen sind zudem die hohen Kosten derartiger Beschichtungssysteme zu nennen.

Aus der EP 1 616 939 A1 ist ein Zellkultursubstrat bekannt, das mit einem hydrophoben bindungsabsorbierenden Polymer beschichtet ist, das ein hydrophobes lineares Gerüst und eine funktionelle Gruppe aufweist, die mit einem Protein oder einem Peptid in einem Molekül reagieren kann.

In der US 2012/052580 A1 ist ein mit einem Polymer beschichteter Zellkulturträger beschrieben. Dabei weist das Polymer Einheiten auf, welche jeweils eine Carboxylgruppe und eine Peptid-modifizierte Gruppe enthalten. Darin ist mindestens ein Peptid mit 5 bis 50 Aminosäureresten enthalten.

In der US 2010/304427 A1 ist ein Substrat für eine Zellkultur und zellbasierte Analysen beschrieben. Dieses Substrat umfasst einen Träger, eine Verbindungsschicht in Form eines Aminoalkylsilans oder Derivaten davon, die an den Träger befestigt ist, sowie eine auf die Verbindungsschicht aufgebrachte synthetische Polymerschicht. Dabei umfasst die synthetische Polymerschicht eine Vielzahl von ionisierbaren hydrophilen Gruppen, ionisierbaren hydrophoben Gruppen oder Kombinationen davon. Des Weiteren beschreibt die US 2010/304427 A1 ein Verfahren zur Herstellung des Substrats für die Zellkultur und zellbasierte Analysen. Dieses Verfahren umfasst die Anbringung der Verbindungsschicht am Träger sowie die Anbringung der synthetischen Polymerschicht, zum Beispiel in Form von Polyethylen-Maleinsäureanhydrid, auf die Verbindungsschicht.

Die der Erfindung zugrundeliegende Aufgabe besteht in der Bereitstellung eines einfach zu realisierenden bioaktiven, insbesondere zellinstruktiven Beschichtungssystems. Das Beschichtungsmaterial sollte bei den üblichen Aufgabemengen gegenüber den Zellen keine toxische Wirkung entfalten und eine ausreichende Schichtstabilität bei der Behandlung mit Spüllösungen oder komplexen Zellkulturmedien, insbesondere Pufferlösungen oder serumhaltigen Medien, aufweisen.

Die Aufgabe wird mit einem Verfahren zur Beschichtung von Zellkulturträgern aus Kunststoff mit den Merkmalen von Anspruch 1 gelöst. Das dabei verwendete bioaktive Beschichtungsmaterial ist für Zellkulturträger aus Kunststoff, insbesondere Zellkultureinwegmaterialien, zum Beispiel aus Polystyren (TCP) als Kunststoff geeignet. Es umfasst ein Polymerkonjugat aus jeweils einem polymeren Ankermolekül mit oberflächenaktiven Ankergruppen einerseits und aus jeweils einem oder mehreren biologisch aktiven Molekülen andererseits. Erfindungsgemäß ist das Ankermolekül ein amphiphiles Molekül mit einem hydrophoben Teil aus Styren- oder Isobuteneinheiten und einem hydrophilen Teil aus Maleinsäureanhydrid- oder Maleinsäureeinheiten.

Als bioaktives Beschichtungsmaterial wird dabei bevorzugt ein zellinstruktives Beschichtungsmaterial, insbesondere zelladhäsives Beschichtungsmaterial, verwendet. Es können demgegenüber auch bioaktive Beschichtungsmaterialien verwendet werden, die antiadhäsiv wirkende Moleküle, zum Beispiel Polyethylenglykol (PEG), enthalten, welche die Zelladhäsion vermindern und auf diese Weise ebenfalls zellinstruktiv wirken. Durch die Auswahl von diesem Beschichtungsmaterial lassen sich Zellschicksalsentscheidungen, insbesondere aber die Zelladhäsion, auf nahezu beliebigen Zellkulturartikeln, insbesondere Zellkultur-Einwegartikeln, individuell und effektiv durch den Anwender steuern. Mit diesem Konzept werden insgesamt wertvolle neue Optionen für High-Throughput-Diagnostik, Stammzell-Biotechnologie und regenerative Therapien eröffnet. In Erweiterung des zuvor beschriebenen Standes der Technik wurde eine vollsynthetische Beschichtung entwickelt, die die Vorteile der zuvor beschriebenen Verfahren vereint, ohne auf biologische Quellen zurückgreifen zu müssen. Als zusätzlicher Vorteil ist zu nennen, dass das Aufbringen einer adhäsiven Beschichtung als einfacher Adsorptionsprozess aus wässrigen Lösungen direkt beim Endanwender erfolgen kann.

Die bioaktiven Beschichtungsmaterialien können neben zellinstruktiv wirkenden Molekülen auch antimikrobielle, das heißt biozid oder biostatisch wirkende Moleküle als biologisch aktive Moleküle enthalten. Bevorzugt sind dabei insbesondere antibakterielle Beschichtungsmaterialien.

Gemäß einer Ausführungsform der Erfindung ist das biologisch aktive Molekül durch eine enzymatisch spaltbare Peptidsequenz mit dem Ankermolekül verbunden.

Vorzugsweise ist das biologisch aktive Molekül dabei durch eine enzymatisch spaltbare Peptidsequenz, welche durch die Klasse der Peptidasen, beispielsweise der Metalloproteasen, der Cystein-, Aspartyl-, Serin- und Threonylproteasen gespaltet wird, mit dem Ankermolekül verbunden.

Gemäß einer weiteren Ausführungsform der Erfindung ist das biologisch aktive Molekül durch eine enzymatisch spaltbare Peptidsequenz, bestehend aus 5 bis 25 Aminosäureresten, welche beispielsweise die Aminosäuresequenz GPQGIWGQ enthält, mit dem Ankermolekül verbunden.

Gemäß einer weiteren Ausführungsform der Erfindung ist das biologisch aktive Molekül ein Signalmolekül, vorzugsweise aus der Gruppe der Neurotransmitter, Hormone, Zytokine, Wachstumsfaktoren oder Chemokine.

Gemäß einer weiteren bevorzugten Ausgestaltung der Erfindung ist das biologisch aktive Molekül ein Peptid, welches Zellschicksalsentscheidungen beeinflussen kann. Gemäß einer besonders bevorzugten Ausführungsform ist das biologisch aktive Molekül eine aus einem in der extrazellulären Matrix (ECM) vorkommenden Protein, wie zum Beispiel Kollagen, Fibronektin oder Laminin, abgeleitete Peptideinheit. Dies kann beispielsweise ein Adhäsionspeptid bestehend aus 3 bis 50 Aminosäureresten sein. Das biologisch aktive Molekül ist gemäß einer vorteilhaften Ausführungsform ein Adhäsionspeptid, welches die Aminosäuresequenz RGD oder RGDSP oder SIKVAV oder YIGSR oder EIDGELT oder EIKLLIS oder RKRLQVQLSIRT enthält.

Zur Kontrolle von Zellschicksalsentscheidungen, insbesondere zur Vermittlung der Adhäsion, werden die kurzen Peptideinheiten, die aus ECM-Proteinen abgeleitet sind, welche natürlicherweise zelluläre Rezeptoren binden, durch Festphasenpeptidsynthese erzeugt. Diese Peptideinheiten werden dann kovalent an die synthetischen polymeren Ankermoleküle gekoppelt, die ausgeprägt grenzflächenaktiv sind. Diese Ankermoleküle weisen dementsprechend einen hydrophoben, das heißt grenzflächenaffinen Teil sowie einen hydrophilen, das heißt die Wasserlöslichkeit vermittelnden Teil aus, das heißt sie sind amphiphil. Dadurch können derartige Moleküle effektiv aus wässrigen Lösungen an Kunststoffoberflächen verschiedenster Art abgeschieden werden. Dies kann beispielsweise direkt beim Endanwender erfolgen. Durch die Möglichkeit, Polymeranker mit unterschiedlich starker Oberflächenaffinität zu verwenden, welche zudem mit verschiedenen Peptidsequenzen oder Signalmolekülen gekoppelt sein können, lassen sich somit Zellschicksalsentscheidungen, insbesondere aber die Adhäsion unterschiedlicher Zelltypen, definiert und kostengünstig modulieren.

Vorteilhaft können dabei Ankermoleküle verwendet werden, die entlang der Polymerkette mehrere Gruppen für eine Reaktion mit funktionellen Peptiden oder Signalmolekülen aufweisen. In einer Ausführungsform ist das Ankermolekül ein Molekül eines Copolymers mit alternierenden Isobuten- und Maleinsäureanhydrideinheiten oder alternierenden Isobuten- und Maleinsäureeinheiten. Ein solches Ankermolekül hat vorzugsweise eine molare Masse von 4.000 g/mol.

In einer besonders vorteilhaften Ausführungsform der Erfindung ist das Ankermolekül ein Copolymer mit alternierenden Styren- und Maleinsäureanhydrideinheiten oder alternierenden Styren- und Maleinsäureeinheiten, vorzugsweise mit einer molaren Masse von 20.000 bis 25.000 g/mol.

Ein weiterer Aspekt betrifft einen Zellkulturträger aus Polystyren (TCP), der mit einem erfindungsgemäß verwendeten bioaktiven Beschichtungsmaterial in einer der oben beschriebenen Varianten beschichtet ist, wobei die Verbindung des bioaktiven Beschichtungsmaterials mit dem Zellkulturträger gemäß der Erfindung adsorptiv über die hydrophoben Teile der Ankermoleküle ausgebildet ist.

Das bioaktive Beschichtungsmaterial kann sowohl vom Hersteller der Zellkulturträger als auch vom Endanwender zur Beschichtung von Zellkulturträgern aus Kunststoffeinwegmaterialien, zum Beispiel aus Polystyren (TCP) verwendet werden.

Die Vorteile der Erfindung lassen sich folgendermaßen zusammenfassen:
Die bioaktive Beschichtung
- ermöglicht eine ausreichend stabile Verankerung/Adhäsionsvermittlung auf Standardzellkulturartikeln,
- ist durch einen einfachen Adsorptionsprozess aufzubringen,
- ruft keine zytotoxischen Wirkungen hervor und
- zeichnet sich als vollsynthetisches System durch gut definierte, gleichbleibende Eigenschaften aus.

Der Kundennutzen besteht gegenüber dem Stand der Technik vor allem in einem deutlichen Performancegewinn. Dieser wird durch die definierte gleichbleibende Qualität der vollsynthetischen Beschichtungen erreicht, die in ihrer Bindungsstärke zu den Kunststoffoberflächen gezielt einstellbar sind und zum Beispiel eine zellinstruktive, insbesondere adhäsionsvermittelnde Funktionalität von ECM-Proteinen bieten können. Die Kombination unterschiedlicher bioaktiver Moleküle, insbesondere verschiedener Adhäsionsliganden, zum Beispiel aus Fibronektin und Laminin abgeleiteter Peptidsequenzen, erlaubt die Anpassung an die Anforderungen unterschiedlicher Zellkulturen und begründet einen weiteren wesentlichen Qualitätsvorteil gegenüber den bestehenden bioaktiven Beschichtungen. Der wichtigste Vorteil besteht in der Nutzung optimaler Ankerpolymere, welche selbst in komplex zusammengesetzten biologischen Medien nach der einfachen Adsorption aus wässrigen Lösungen stabil auf den Kunststoffoberflächen verbleiben. Als wichtigster Kundennutzen sind zusätzlich wirtschaftliche Erwägungen anzuführen. Gegenüber den zumeist sehr preisintensiven Beschichtungen der etablierten Hersteller wird eine deutlich preisgünstigere Alternative geboten, welche die Endanwender zeitsparend direkt auf den bei ihnen bereits etablierten, deutlich kostengünstigeren Standard-Zellkulturprodukten aufbringen können. Die Grundlage hierfür liegt in dem gegenwärtigen Preisniveau der Einwegartikel. Unbeschichtete Zellkulturplatten erzielen derzeit einen Preis von 2,20 - 3,00 Euro je Platte, während die bioaktiv beschichteten Platten in einer Preisspanne zwischen 16,00 bis 35,00 Euro pro Platte verkauft werden. Die bisher einzigen synthetischen Beschichtungen mit kovalent an Oberflächen gebundenen Adhäsionspeptiden, die CORNING® Synthemax™- Beschichtungen, liegen dabei mit 27,00 bis 34,00 Euro am oberen Ende der Preisspanne. Zudem können adhäsiv beschichtete Oberflächen auch Nutzern zugänglich gemacht werden, die mit Zellkulturprodukten arbeiten, die derzeit nicht beschichtet angeboten werden.

Mit der Bereitstellung gebrauchsfertiger wässriger Lösungen zur Adsorption der bioaktiven Beschichtungen kann ein deutlicher Preisvorteil beim Endanwender realisiert werden.

Weitere Einzelheiten, Merkmale und Vorteile der Erfindung ergeben sich aus der nachfolgenden Beschreibung von Ausführungsbeispielen mit Bezugnahme auf die zugehörigen Zeichnungen. Es zeigen:
- **Fig. 1:**: eine schematische Übersicht zu einem bioaktiven, zelladhäsiven Beschichtungssystem,
- **Fig. 2:**: die Auswertung von Messungen zur Schichtstabilität,
- **Fig. 3:**: die Auswertung von Messungen zur Polymertoxizität,
- **Fig. 4:**: die Analyse der initialen Zelladhäsion durch die Bestimmung der Anzahl der adhärenten Zellen an verschiedenen modifizierten Oberflächen und
- **Fig. 5:**: die Analyse der initialen Zelladhäsion durch die Bestimmung der Elongation der Zellen an verschiedenen modifizierten Oberflächen.

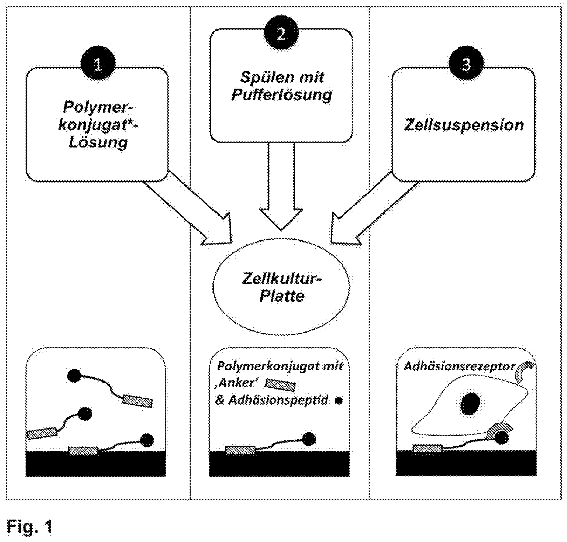

Die **Fig. 1** zeigt eine schematische Übersicht eines bioaktiven, im vorliegenden Fall eines zelladhäsiven Beschichtungssystems als Beispiel für ein zellinstruktives Beschichtungssystem, das für *in situ* Coatings geeignet ist. Im Schritt (1) werden die Polymerkonjugate durch Adsorption aus der Lösung aufgebracht. Im Schritt (2) erfolgt das Spülen der Zellkulturträger mit Phosphatgepufferter Kochsalzlösung (PBS) und somit die Entfernung der nicht festhaftenden Polymerkonjugate. In Schritt (3) erfolgt die Kultur der Zellen auf dem Träger. Wesentlicher Teil des Beschichtungssystems ist das Polymerkonjugat. Der Aufbau dieses Polymerkonjugats erfolgt schrittweise nach bekannten Verfahren. Zur Vermittlung der Adhäsion werden kurze Peptideinheiten, die aus ECM-Proteinen abgeleitet sind, welche natürlicherweise zelluläre Rezeptoren binden, durch Festphasenpeptidsynthese erzeugt. Diese Peptideinheiten werden dann kovalent an die synthetischen polymeren Ankermoleküle gekoppelt. Die Ankermoleküle sind ausgeprägt grenzflächenaktiv und können dadurch effektiv an Kunststoffoberflächen verschiedenster Art, insbesondere aber an Polystyren, abgeschieden werden. Das Aufbringen dieser Peptid-Polymerkonjugate kann dabei direkt beim Endanwender durch Adsorption aus wässrigen Lösungen erfolgen, wie in der **Fig. 1** gezeigt. Durch die Möglichkeit, Polymeranker mit unterschiedlich starker Oberflächenaffinität zu verwenden, welche zudem mit verschiedenen Peptidsequenzen gekoppelt sein können, lässt sich somit erstmals die Adhäsion unterschiedlicher Zelltypen definiert und kostengünstig modulieren.

Das Ankermolekül ist ein amphiphiles Molekül mit einem hydrophoben Teil aus Styren- oder Isobuteneinheiten und einem hydrophilen Teil aus Maleinsäureanhydrid- oder Maleinsäureeinheiten. Zur Herstellung des Polymerkonjugates können die Carboxylgruppen des Ankermoleküls zunächst auf bekanntem Wege mit Maleimid mittels Carbodiimid-Chemie funktionalisiert werden. Im Anschluss daran kann die Ankopplung der Peptideinheiten über eine Michael-Addition erfolgen, beispielsweise die Ankopplung von RGDSP über ein Cystein in der Sequenz, die komplett aus NH2-CWGRGDSP-CONH2 aufgebaut ist. Das amphiphile Ankermolekül Polymer 1 besteht aus einem alternierenden Copolymer aus Isobuten und Maleinsäureanhydrid, wobei die Isobuteneinheiten den hydrophoben Teil und die MaleinsäureanhydridEinheiten den hydrophilen Teil bilden. Das amphiphile Ankermolekül Polymer 2 besteht aus einem amphiphilen Ankermolekül, dass aus einem alternierenden Copolymer aus Styren und Maleinsäureanhydrid gebildet wird, wobei die Styreneinheiten den hydrophoben Teil und die Maleinsäureanhydrideinheiten den hydrophilen Teil bilden.

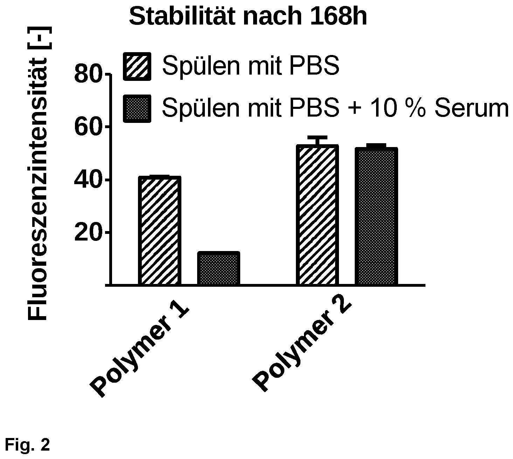

Die **Fig. 2** zeigt die Retention der Polymere 1 und 2 auf Zellkulturplastik aus Polystyren (TCP) nach dem Spülen mit Phosphatgepufferter Kochsalzlösung (PBS) und mit einer Mischung aus 10 % Serum, gelöst in Phosphatgepufferter Kochsalzlösung (PBS) für jeweils 168 Stunden. Als Indikator der Polymere an der Oberfläche wird ein Fluoreszenzsignal erfasst, wobei die Polymere zu diesem Zweck fluoreszenzmarkiert sind.

Dabei zeigte das Polymer 2 eine konstante Oberflächenverankerung sowohl beim Spülen mit PBS als auch beim Spülen mit der Mischung aus PBS und 10 % Serum. Polymer 1 zeigte eine relativ langsame Desorption beim Spülen mit PBS, wobei die Desorption bei Anwendung der Mischung aus PBS und 10 % Serum verstärkt wird. Durch diese langsame beziehungsweise je nach Spülmittel (bzw. anwendungsrelevanten Zellkulturmedium) abgestufte Desorption besteht die Möglichkeit, für Zellanwendungen mit sehr unterschiedlichen Erfordernissen entweder eine zeitlich modellierbare oder eine gleichbleibend stabile Verankerung von Adhäsionsliganden einzurichten.

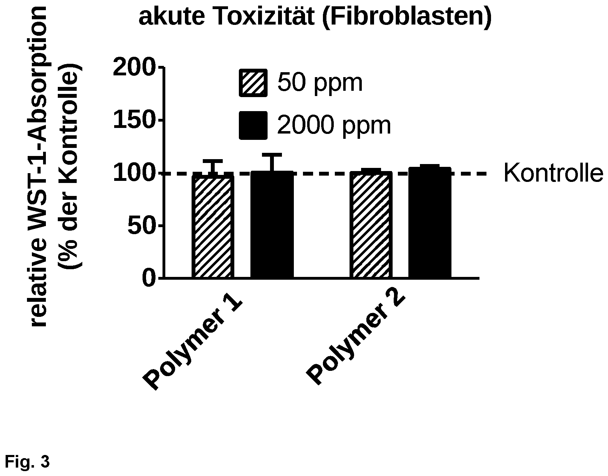

Die **Fig. 3** zeigt die Ergebnisse eines Tests der akuten Zelltoxizität nach Voradsorption der Polymere 1 oder 2 auf Zellkulturplastik aus Polystyren (TCP) durch die Bestimmung der Stoffwechselaktivität von Fibroblasten im Vergleich zur unbehandelten Zellkulturoberfläche. Die Bestimmung der Zelltoxizität erfolgt mittels WST-1-Assay, wobei WST die Abkürzung für "water soluble tetrazolium" ist. Der WST-1-Assay dient zum Nachweis einer intakten Atmungskette in Zellen. Lebende Zellen mit einem intakten mitochondrialen Succinat-Tetrazolium-Dehydrogenase-System bewirken eine enzymatische Umsetzung des schwach rot gefärbten Tetrazoliumsalzes WST-1 (4-[3-(4-lodophenyl)-2-(4-nitrophenyl)-2H-5-tetrazolio]-1,3-Benzol-Disulfonat) in das dunkelrote Formazan. Dieser Farbumschlag kann in einem Spektralphotometer photometrisch gemessen und ausgewertet werden. In **Fig. 3** ist die spektralphotometrische WST-Absorption nach Voradsorption der Polymere 1 oder 2 jeweils im Vergleich zur Absorption in Gegenwart einer unbehandelten Zellkulturoberfläche, die einem Wert von 100 % entspricht, als relativer Wert angegeben.

Wie aus der **Fig. 3** hervorgeht, zeigen weder das Polymer 1 noch das Polymer 2 in adsorbierter Form in einer für die Anwendung relevanten Darreichung von 50 beziehungsweise 2000 ppm toxische Wirkung.

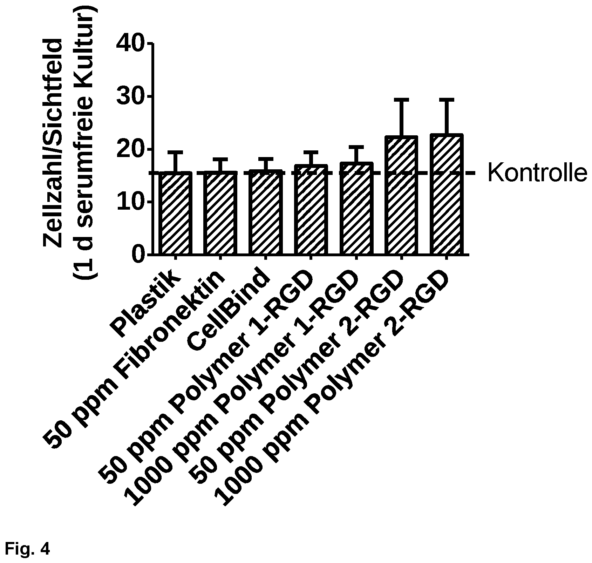

Die **Fig. 4** zeigt die Ergebnisse der Analyse der Zelladhäsion von humanen Endothelzellen (HUVEC) an den durch Voradsorption der mit RGD konjugierten Polymere 1 und 2 (Polymer 1-RGD und Polymer 2-RGD) modifizierten Oberflächen mittels Zählung. Dabei erfolgt eine Untersuchung potentiell adhäsionsvermittelnder Effekte der mit RGD konjugierten Polymere 1 und 2. Die experimentellen Untersuchungen erfolgten nach einer 24-stündigen serumfreien Kultur.

Als Negativkontrolle diente dabei ein Plastik-Zellkulturträger. Als Positivkontrolle diente Fibronektin, voradsorbiert aus einer Lösung mit einer Konzentration von 50 ppm. Eine weitere Vergleichsanalyse wurde auf einem Zellkulturträger aus plasmabehandelter Plastik (Corning®CellBIND®) durchgeführt. Ein Teil der Untersuchungen erfolgte auf einer jeweils mit 50 ppm beziehungsweise mit 1000 ppm Konjugat aus Polymer 1 und RGD durch Adsorption vorausgerüsteten Zellkulturträgeroberfläche. Ein weiterer Teil der Untersuchungen erfolgte auf einer jeweils mit 50 ppm beziehungsweise mit 1000 ppm Konjugat aus Polymer 2 und RGD durch Adsorption vorausgerüsteten Zellkulturträgeroberfläche.

In serumfreier Kultur erhöht die Präsenz der mit RGD konjugierten Polymere 1 und 2 (Polymer 1-RGD und Polymer 2-RGD) die Anzahl adhärenter Zellen, wobei diese Erhöhung bei Anwendung vom Konjugat aus Adhäsionsligand und Polymer 2 (Polymer 2-RGD) noch viel stärker ausfällt als bei Anwendung vom Konjugat aus Adhäsionsligand und Polymer 1 (Polymer 1-RGD).

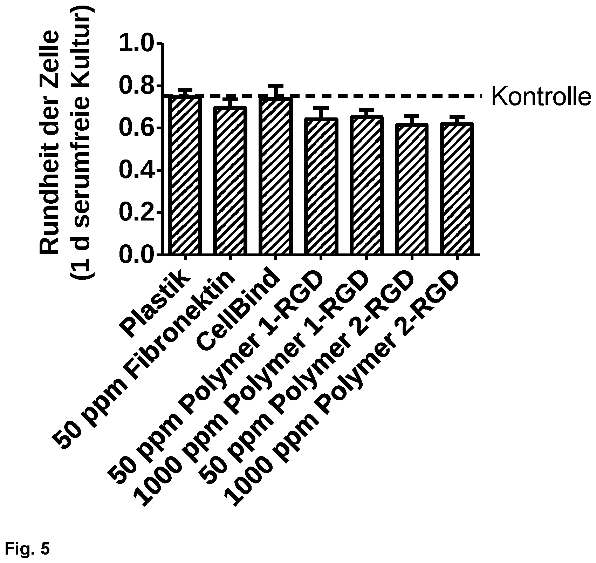

Die **Fig. 5** zeigt die Morphologie, beschrieben als Aspektverhältnis, humaner Endothelzellen nach Voradsorption der Konjugate aus Adhäsionsligand und der beiden Polymere 1 und 2 (Polymer 1-RGD und Polymer 2-RGD) auf Zellkulturplastik aus Polystyren (TCP). Die biologisch gewünschte Elongation der Endothelzellen wird durch ein niedriges Aspektverhältnis ausgedrückt. Dies ist das Verhältnis der kurzen zur langen Achse, der Koeffizient 1 entspricht einer runden Zelle, ein Koeffizient < 1 entspricht der gewünschten länglichen Morphologie. Die Messungen wurden jeweils nach 24 Stunden serumfreier Kultur durchgeführt.

In serumfreier Kultur erhöht die Präsenz der mit RGD konjugierten Polymere 1 und 2 (Polymer 1-RGD und Polymer 2-RGD) die Elongation adhärenter Zellen sogar effektiver als bei der Positivkontrolle mit Fibronektin. Das heißt, mit beiden Konjugaten werden höhere Elongationswerte erreicht als mit den Vergleichsproben auf Plastik, mit 50 ppm Fibronektin und mit dem Zellkulturträger aus plasmabehandelter Plastik CellBind. Dabei wurden für das Konjugat aus Adhäsionsliganden mit Polymer 2 (Polymer 2-RGD) noch niedrigere Rundheitswerte gemessen als für das Konjugat aus Adhäsionsliganden mit Polymer 1 (Polymer 1-RGD). Die Rundheitswerte, die für die unterschiedlichen Darreichungsmengen von 50 ppm beziehungsweise 1000 gemessen wurden, unterscheiden sich nicht wesentlich.

## Patentansprüche

1. Verfahren zur Beschichtung von Zellkulturträgern aus Kunststoff, bei dem ein bioaktives Beschichtungsmaterial, umfassend ein Polymerkonjugat aus jeweils einem polymeren Ankermolekül mit oberflächenaktiven Ankergruppen einerseits und aus jeweils einem oder mehreren biologisch aktiven Molekülen andererseits, wobei das Ankermolekül ein amphiphiles Molekül mit einem hydrophoben, grenzflächenaffinen Teil aus Styren- oder Isobuteneinheiten und einem hydrophilen, die Wasserlöslichkeit des Ankermoleküls vermittelnden Teil aus Maleinsäureanhydrid- oder Maleinsäureeinheiten ist und das jeweils konjugierte biologisch aktive Molekül ein PEG-Molekül, ein antimikrobielles Molekül, ein Signalmolekül aus der Gruppe der Zytokine, Wachstumsfaktoren oder Chemokine oder ein Peptid mit 3 bis 50 Aminosäureresten ist, aus wässriger Lösung abgeschieden wird, und wobei die Verbindung des bioaktiven Beschichtungsmaterials mit dem Zellkulturträger adsorptiv über die hydrophoben Teile der Ankermoleküle ausgebildet wird.

2. Verfahren nach Anspruch 1, **dadurch gekennzeichnet, dass** das biologisch aktive Molekül durch eine enzymatisch spaltbare Peptidsequenz mit dem Ankermolekül verbunden ist.

3. Verfahren nach Anspruch 2, **dadurch gekennzeichnet, dass** das biologisch aktive Molekül durch eine enzymatisch spaltbare Peptidsequenz, welche durch die Klasse der Peptidasen, beispielsweise der Metalloproteasen, der Cystein-, Aspartyl-, Serin- und Threonylproteasen gespaltet wird, mit dem Ankermolekül verbunden ist.

4. Verfahren nach Anspruch 2, **dadurch gekennzeichnet, dass** das biologisch aktive Molekül durch eine enzymatisch spaltbare Peptidsequenz, bestehend aus 5 bis 25 Aminosäureresten, welche beispielsweise die Aminosäuresequenz GPQGIWGQ enthält, mit dem Ankermolekül verbunden ist.

5. Verfahren nach einem der Ansprüche 1 bis 4, **dadurch gekennzeichnet, dass** das biologisch aktive Molekül ein Adhäsionspeptid mit 3 bis 50 Aminosäureresten ist.

6. Verfahren nach einem der Ansprüche 1 bis 5, **dadurch gekennzeichnet, dass** das biologisch aktive Molekül ein Peptid ist, das aus einem in der extrazellulären Matrix vorkommenden Protein abgeleitet ist.

7. Verfahren nach einem der Ansprüche 1 bis 6, **dadurch gekennzeichnet, dass** das biologisch aktive Molekül ein Peptid ist, das aus Kollagen, Fibronektin oder Laminin abgeleitet ist.

8. Verfahren nach Anspruch 5, **dadurch gekennzeichnet, dass** das biologisch aktive Molekül ein Adhäsionspeptid ist, welches die Aminosäuresequenz RGD oder RGDSP oder SIKVAV oder YIGSR oder EIDGELT oder EIKLLIS oder RKRLQVQLSIRT enthält.

9. Verfahren nach einem der Ansprüche 1 bis 8, **dadurch gekennzeichnet, dass** das Ankermolekül ein Molekül eines Copolymers mit alternierenden Isobuten- und Maleinsäureanhydrideinheiten oder alternierenden Isobuten- und Maleinsäureeinheiten ist.

10. Verfahren nach Anspruch 9, **dadurch gekennzeichnet, dass** das Ankermolekül eine molare Masse von 4.000 g/mol aufweist.

11. Verfahren nach einem der Ansprüche 1 bis 8, **dadurch gekennzeichnet, dass** das Ankermolekül ein Copolymer mit alternierenden Styren- und Maleinsäureanhydrideinheiten oder alternierenden Styren- und Maleinsäureeinheiten ist.

12. Verfahren nach Anspruch 11, **dadurch gekennzeichnet, dass** das Ankermolekül eine molare Masse von 20.000 bis 25.000 g/mol aufweist.

## Claims

1. A method for coating cell culture carriers of plastic in which a bioactive coating material, comprising a polymer conjugate each of a polymer anchor molecule having surface active anchor groups on the one hand and each of one or more biologically active molecules on the other hand, wherein the anchor molecule is an amphiphile molecule with a hydrophobic moiety with interface affinity made of styrene or isobutene units and a hydrophilic moiety imparting the water solubility of the anchor molecule made of maleic acid anhydride or maleic acid units and each conjugated biologically active molecule is a PEG molecule, an antimicrobial molecule, a signal molecule from the group of cytokines, growth factors or chemokines or a peptide with 3 to 50 amino acid residues, is precipitated from aqueous solution, and wherein the bond of the bioactive coating material with the cell culture carrier is adsorptively formed via the hydrophobic moieties of the anchor molecules.

2. The method of claim 1, **characterized in that** the biologically active molecule is bound to the anchor molecule via an enzymatically cleavable peptide sequence.

3. The method of claim 2, **characterized in that** the biologically active molecule is bound to the anchor molecule via an enzymatically cleavable peptide sequence, cleavable by the class of peptidases, for example, metal proteases such as cysteine-, aspartyl-, serine-, and threonyl proteases.

4. The method of claim 2, **characterized in that** the biologically active molecule is bound to the anchor molecule via an enzymatically cleavable peptide sequence consisting of 5 to 25 amino acid residues, which for example includes the amino acid sequence GPQGIWGQ.

5. The method of one of claims 1 to 4, **characterized in that** the biologically active molecule is an adhesion peptide with 3 to 50 amino acid residues.

6. The method of one of claims 1 to 5, **characterized in that** the biologically active molecule is a peptide that is derived from a protein that is present in the extracellular matrix.

7. The method of one of claims 1 to 6, **characterized in that** the biologically active molecule is a peptide derived from collagen, fibronectin or laminin.

8. The method of claim 5, **characterized in that** the biologically active molecule is an adhesion peptide which includes the amino acid sequence RGD or RGDSP or SIKVAV or YIGSR or EIDGELT or EIKLLIS or RKRLQVQLSIRT.

9. The method of one of claims 1 to 8, **characterized in that** the anchor molecule is a molecule of a copolymer with alternating isobutene and maleic acid anhydride units or alternating isobutene and maleic acid units.

10. The method of claim 9, **characterized in that** the anchor molecule has a molar mass of 4000 g/mol.

11. The method of one of claims 1 to 8, **characterized in that** the anchor molecule is a copolymer with alternating styrene- and maleic acid anhydride units or alternating styrene- and maleic acid units.

12. The method of claim 11, **characterized in that** the anchor molecule has a molar mass of 20 000 to 25 000 g/mol.

## Revendications

1. Procédé de revêtement de supports de culture de cellules en matériau synthétique, selon lequel un matériau de revêtement bioactif est séparé d'une solution aqueuse, ledit matériau comprenant un conjugué de polymères composé respectivement d'une molécule d'ancrage polymère avec des groupes d'ancrage tensioactifs d'une part et d'un ou de plusieurs molécules biologiquement actives d'autre part, moyennant quoi la molécule d'ancrage est une molécule amphiphile avec une partie hydrophobe à affinité de surface composée d'unités de styrène ou d'isobutène et une partie hydrophile composée d'unités anhydrides d'acide maléique ou d'unités d'acide maléique facilitant la solubilité dans l'eau de la molécule d'ancrage, et la molécule respectivement conjuguée biologiquement active est une molécule PEG, une molécule antimicrobienne, une molécule de signal issue du groupe des cytokines, des facteurs de croissance ou des chimiokines ou un peptide avec 3 à 50 résidus d'acides aminés, et selon lequel la liaison du matériau de revêtement bioactif avec le support de culture de cellules est formée par adsorption par les parties hydrophobes des molécules d'ancrage.

2. Procédé selon la revendication 1, **caractérisé en ce que** la molécule biologiquement active est liée à la molécule d'ancrage par une séquence de peptides scindable par voie enzymatique.

3. Procédé selon la revendication 2, **caractérisé en ce que** la molécule biologiquement active est liée à la molécule d'ancrage par une séquence de peptides scindable par voie enzymatique qui est scindée par la classe des peptidases, par exemple des métalloprotéases, des cystéines-protéases, des protéases aspartiques, des protéases à sérine et des thréonyl-protéases.

4. Procédé selon la revendication 2, **caractérisé en ce que** la molécule biologiquement active est liée à la molécule d'ancrage par une séquence de peptides scindable par voie enzymatique, composée de 5 à 25 résidus d'acides aminés, qui contient par exemple la séquence d'acides aminés GPQGIWGQ.

5. Procédé selon l'une quelconque des revendications 1 à 4, **caractérisé en ce que** la molécule biologiquement active est un peptide d'adhésion avec 3 à 50 résidus d'acides aminés.

6. Procédé selon l'une quelconque des revendications 1 à 5, **caractérisé en ce que** la molécule biologiquement active est un peptide qui est dérivé d'une protéine présente dans la matrice extracellulaire.

7. Procédé selon l'une quelconque des revendications 1 à 6, **caractérisé en ce que** la molécule biologiquement active est un peptide qui est dérivé du collagène, de la fibronectine ou de la laminine.

8. Procédé selon la revendication 5, **caractérisé en ce que** la molécule biologiquement active est un peptide d'adhésion qui contient la séquence d'acides aminés RGD ou RGDSP ou SIKVAV ou YIGSR ou EIDGELT ou EIKLLIS ou RKRLQVQLSIRT.

9. Procédé selon les revendications 1 à 8, **caractérisé en ce que** la molécule d'ancrage est une molécule d'un copolymère avec des unités anhydrides alternantes d'isobutène et d'acide maléique ou des unités alternantes d'isobutène et d'acide maléique.

10. Procédé selon la revendication 9, **caractérisé en ce que** la molécule d'ancrage présente une masse moléculaire de 4 000 g/mol.

11. Procédé selon l'une quelconque des revendications 1 à 8, **caractérisé en ce que** la molécule d'ancrage est un copolymère avec des unités anhydrides alternantes de styrène et d'acide maléique ou d'unités alternantes de styrène et d'acide maléique.

12. Procédé selon la revendication 11, **caractérisée en ce que** la molécule d'ancrage présente une masse moléculaire de 20 000 à 25 000 g/mol.


### SEQUENCE LISTING

<110> ZetaSCIENCE GmbH
<120> Bioaktives Beschichtungsmaterial
<130> ZS2-14-IP
<150> DE102015109599
   <151> 2015-06-16
<160> 8
<170> PatentIn version 3.5
<210> 1
   <211> 8
   <212> PRT
   <213> Artificial Sequence
<220>
   <223> enzymatisch spaltbare Sequenz
<400> 1
   Gly Pro Gln Gly Ile Trp Gly Gln
   1               5
<210> 2
   <211> 5
   <212> PRT
   <213> Artificial Sequence
<220>
   <223> Aminosaeuresequenz eines Adhaesionspeptids
<400> 2
   Arg Gly Asp Ser Pro
   1               5
<210> 3
   <211> 6
   <212> PRT
   <213> Artificial Sequence
<220>
   <223> Aminosaeuresequenz eines Adhaesionspeptids
<400> 3
   Ser Ile Lys Val Ala Val
   1               5
<210> 4
   <211> 5
   <212> PRT
   <213> Artificial Sequence
<220>
   <223> Aminosaeuresequenz eines Adhaesionspeptids
<400> 4
   Tyr Ile Gly Ser Arg
   1               5
<210> 5
   <211> 7
   <212> PRT
   <213> Artificial Sequence
<220>
   <223> Aminosaeuresequenz eines Adhaesionspeptids
<400> 5
   Glu Ile Asp Gly Glu Leu Thr
   1               5
<210> 6
   <211> 7
   <212> PRT
   <213> Artificial Sequence
<220>
   <223> Aminosaeuresequenz eines Adhaesionspeptids
<400> 6
   Glu Ile Lys Leu Leu Ile Ser
   1               5
<210> 7
   <211> 12
   <212> PRT
   <213> Artificial Sequence
<220>
   <223> Aminosaeuresequenz eines Adhaesionspeptids
<400> 7
   Arg Lys Arg Leu Gln Val Gln Leu Ser Ile Arg Thr
   1               5                   10
<210> 8
   <211> 8
   <212> PRT
   <213> Artificial Sequence
<220>
   <223> Aminosaeuresequenz eines Adhaesionspeptids
<400> 8
   Cys Trp Gly Arg Gly Asp Ser Pro
   1               5